(19) Europäisches Patentamt
European Patent Office
Office européen des brevets

(11) Veröffentlichungsnummer: **0 034 692**
**B1**

# (12) EUROPÄISCHE PATENTSCHRIFT

(45) Veröffentlichungstag der Patentschrift: **08.01.86**

(51) Int. Cl.⁴: **C 12 Q 1/34**, C 08 B 31/12, G 01 N 33/50

(21) Anmeldenummer: **81100139.5**

(22) Anmeldetag: **10.01.81**

## (54) Die Verwendung von Stärkederivaten für Indikatormittel.

(30) Priorität: **19.01.80 DE 3001878**

(43) Veröffentlichungstag der Anmeldung: **02.09.81 Patentblatt 81/35**

(45) Bekanntmachung des Hinweises auf die Patenterteilung: **08.01.86 Patentblatt 86/2**

(84) Benannte Vertragsstaaten: **BE DE FR GB IT NL**

(56) Entgegenhaltungen:
**CH - A - 398 835**
**DE - A - 1 901 517**
**US - A - 3 705 149**

**Die Akte enthält technische Angaben, die nach dem Eingang der Anmeldung eingereicht wurden und die nicht in dieser Patentschrift enthalten sind.**

(73) Patentinhaber: **BEHRINGWERKE AKTIENGESELLSCHAFT, Postfach 1140, D-3550 Marburg/Lahn (DE)**

(72) Erfinder: **Habenstein, Klaus, Dr., Birkenweg 3, D-3551 Lahntal (DE)**
Erfinder: **Kohl, Helmut, Dr., Goethestrasse 32, D-3552 Wetter (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

EP 0 034 692 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von carboxy- oder hydroxyalkylierter Stärke in Indikatorpapieren, -pulver oder -folien.

Gegenstand der Erfindung ist die Verwendung einer Stärkeverbindung der allgemeinen Formel I

$$\underset{\displaystyle (OR)_{3n}}{RO\text{–}[\text{Glucoserest}]_n\text{–}OR} \qquad (I)$$

worin n = 100–12 500 und R Wasserstoff, eine Carboxyalkyl- oder Hydroxyalkylgruppe mit 1–5 Kohlenstoffatomen im Alkylrest und gegebenenfalls eine nachweisbare Molekülgruppe bedeuten, wobei die (3 n + 2) R-Reste statistisch verteilt sind, jedoch so, dass mindestens ein R eine Carboxy- oder Hydroxyalkylgruppe ist, für Indikatorpapiere, -pulver oder -folien.

Bevorzugt wird eine Stärkeverbindung gemäss Formel I verwendet, in der n = 350–12 500 und R Wasserstoff, eine Carboxy- oder Hydroxyalkylgruppe mit 1–3 Kohlenstoffatomen im Alkylrest und gegebenenfalls eine nachweisbare Molekülgruppe sind.

Besonders bevorzugt ist die Verwendung einer Stärkeverbindung gemäss Formel I, in der n = 600–6000 und R Wasserstoff, eine Carboxy- oder Hydroxyalkylgruppe mit 1–2 Kohlenstoffatomen im Alkylrest und gegebenenfalls eine nachweisbare Molekülgruppe sind.

Der Begriff «nachweisbare Molekülgruppe» umfasst chromophore, chromogene, fluoreszierende oder radioaktive Gruppierungen.

Die Verbindungen der Formel I werden von Stärke hydrolysierenden Endohydrolasen hydrolytisch gespalten. Sie können deshalb als Substrate zum Nachweis und zur Bestimmung solcher Hydrolasen verwendet werden.

Da weiterhin gefunden wurde, dass jene löslichen Verbindungen durch Trocknung auf einem Trägermaterial unlöslich werden, sind diese auch zur Herstellung nicht blutender trägergebundener Indikatoren verwendbar.

Die Diagnose einiger lebensbedrohender Erkrankungen ist über die Bestimmung der Serum- oder Urinamylaseaktivität möglich. Es besteht daher ein Bedürfnis für einfache und schnell durchführbare Tests, die mit guter Reproduzierbarkeit diese Gruppe der Polysaccharide hydrolysierenden Endohydrolasen nachzuweisen gestatten.

Es sind Methoden zur Bestimmung solcher Enzyme bekannt, die darauf beruhen, dass der durch Abbau von Stärke gebildete Zucker bestimmt wird.

Andere Verfahren messen die Menge an Farbstoff oder andere Molekülgruppen, die durch die Enzymwirkung aus Stärke abgespalten werden, an welche ein Farbstoff oder andere Molekülgruppen gebunden sind.

So ist ein Verfahren bekannt, das als Substrat eine «Farb-Stärke» benutzt (GB-PS 1 489 059 und L. Fridhandler, J.E. Berk and S. Take, Digestive Diseases (1979) 15 (11), 1039). Hier werden in mehreren Arbeitsschritten wasserlösliche, kolloidale Farbstärkekomplexe auf saugfähige Träger fixiert, indem nach einer ersten Imprägnierung das Substrat auf dem Träger niedergeschlagen wird und in einem weiteren Schritt der Träger von dem überschüssigen, löslichen Substrat gereinigt wird. Es liegt auf der Hand, dass diese drei Arbeitsschritte aufwendig sind und aufgrund der Wechselwirkung «Fixierung – Reinigung» unter anderem bedingt durch Mitreisseffekte zu schwierig reproduzierbaren Testen führen muss.

In weiteren Verfahren werden als Substrate verwendet: Eine wasserlösliche «Farb-Stärke», die aus einer wasserlöslichen Stärke oder Stärkekomponente und einem kovalent an diese gebundenen Farbstoff besteht (US-PS 3 579 322; GB-PS 1 167 083), eine wasserunlösliche «Farb-Stärke», die aus einer wasserunlöslichen Stärke oder Stärkekomponente und einem kovalent an diese gebundenen Farbstoff (H. Rinderknecht, P. Wilding und B.J. Haverback, Experientia 23 (1967), 805 und DE-AS 1 940 869) oder aus einer wasserlöslichen Stärke oder Stärkekomponente und kovalent an diese gebundenen nachweisbaren Molekülgruppen, wobei die Stärke durch Vernetzung wasserunlöslich gemacht wurde (DE-OS 1 901 517, Jap. Kok. 7 521 789, 7 557 697), besteht.

Es bereitet weiter grosse Probleme, einen wasserunlöslichen Farbstärkekomplex in einer geeigneten Form auf einen Reagenzträger aufzutragen, da dieser dann schon bei der Papierherstellung in das Papier eingearbeitet werden muss.

Die Fixierung löslicher Farb-Stärke auf einem damit vorgetränkten Träger erfordert zusätzliche Arbeitsschritte, wobei ausserdem ein teilweises Ausbluten nicht verhindert werden kann.

Der vorliegenden Erfindung lag daher zunächst die Aufgabe zugrunde, ein problemlos und gleichmässig verarbeitbares Substrat für ein einfaches zuverlässiges Verfahren zum Nachweis und zur Bestimmung von Endohydrolasen zu entwickeln.

Diese Aufgabe wird mit einer carboxy- oder hydroxyalkylierten Stärke, an die eine nachweisbare Molekülgruppe gebunden sein kann, gelöst, wobei diese Stärke ein Substrat für Stärke hydrolysierende Endohydrolasen ist.

Es wurde nämlich überraschend gefunden, dass carboxy- oder hydroxy-alkylierte Stärke sowie deren mit nachweisbaren Molekülgruppen derivatisierte Analoga von Stärke hydrolysierenden Endohydrolasen angegriffen werden, eine gute Löslichkeit in wässrigen und polaren aprotischen Lösungsmitteln besitzen und, in wässriger Lösung auf natürliche saugfähige Trägermaterialien aufgetragen, durch einfache Trocknung in nahezu wasserunlöslicher, jedoch quellbarer Form fixiert werden können.

Diese Eigenschaften, die in ihrer Summe weder von natürlichen Stärken oder Stärkeprodukten, noch von anderen Stärkederivaten erreicht werden, machen Carboxyalkyl- und Hydoxyalkyl-Stärken, besonders wenn sie kovalent mit nachweisbaren Molekülgruppen verbunden sind, zu einer geeigneten Substratgrundlage für Indikatorpapiere, besonders aber für diagnostische Mittel zum

Nachweis von Endohydrolasen. Zum Nachweis und zur Bestimmung von Endohydrolasen kann die nachweisbare Molekülgruppe fehlen.

In einer bevorzugten Ausführungsform eines Mittels unter Verwendung einer Verbindung der Formel I besteht dieses aus einem natürlichen saugfähigen Trägermaterial, beispielsweise Cellulose, welches das erfindungsgemässe Substrat und gegebenenfalls ein Puffersalz, einen Accelerator oder Inhibitor und ein Detergenz enthält. Das saugfähige, imprägnierte Trägermaterial wird hierbei so auf einem Reagenzträger angeordnet, dass bei Anwesenheit von Amylase die freigesetzte chromogene Verbindung durch einen einfachen chromatographischen Schritt nachgewiesen werden kann.

Besonders geeignete Stärken sind corboxyalkylierte oder hydroxyalkylierte Stärken vom Typ Amylose, Amylopektin oder Dextrine, die kovalent mit nachweisbaren Molekülgruppen verbunden sein können.

Nachweisbare Molekülgruppen können chemisch gebundene Farbstoffe sein, wie zum Beispiel Remazol®-, Cibachron®-, Drimaren®-, Procion®- oder Lewafix®-Farbstoffe. Nachweisbare Molekülgruppen können aber auch chemisch gebundene Verbindungen sein, die zusätzlich chemisch nachweisbare Gruppierungen aufweisen.

Bei der kovalenten Anbindung der nachweisbaren Molekülgruppen kann die Reaktivität der in Stärke vorhandenen Hydroxylgruppen zu Verätherung oder Veresterung genutzt werden. Beispielsweise können Reaktivfarbstoffe (insbesondere Remazol-Farbstoffe) mit den üblichen Papier- oder Baumwollfärbeverfahren an Carboxymethyl- oder Hydroxyalkyl-Stärke gebunden werden. Mit den in diesen Farbstofftypen zur kovalenten Anbindung benutzten Reaktivgruppierungen (siehe dazu DE-AS 1 293 362, Seite 1 sowie J. Panchartek et al., Coll. Czech., Chem. Commen. (1960) 25, 2783–2799) können ebensogut andere chromogene Gruppen oder fluoreszierende oder Radiotope enthaltende Molekülreste an die den Verbindungen der Formel I zugrunde liegende Stärke gebunden werden.

Die Wahl von Puffersalzen ist prinzipiell unkritisch, sofern die dem Fachmann geläufigen Unverträglichkeiten des jeweiligen Systems beachtet werden (z.B. Hemmung der alpha-Amylase durch EDTA oder Citrat und Salicylsäure). Andererseits können solche Wechselwirkungen zur Einstellung des Systems (z.B. der Nachweisgrenzen) genutzt werden. Der pH-Wert ist in Grenzen variabel und wird sinnvollerweise in dem für das jeweilige Enzym als optimal erkannten Bereich liegen (z.B. pH 6,9–7,2 für alpha-Amylase, pH 4,4–4,6 für beta-Amylase). Als Acceleratoren sind Natriumhalogenide, speziell Natriumchlorid, in Konzentrationen von 10–100 mmol/l bekannt.

Beispiele für Inhibitoren wurden bereits erwähnt (EDTA, Citrate, Salizylsäure). Als weitere, sehr stark wirksame Inhibitoren sind einige aus Getreidesamen gewonnene Proteine bekannt (DE-AS 2 003 934).

Zur Herstellung einer Verbindung der Formel I kann beispielsweise das in US-PS 2 599 620, Beispiele 1 und 2, beschriebene Verfahren zur Carboxymethylierung von Polysacchariden verwendet werden. Hydroxyalkylstärke ist im Handel erhältlich.

Nachweisbare Molekülreste können, wie von H. Rinderknecht, P. Wilding und J.B. Haverback, Experientia 23, 805 (1967) und DE-AS 1 940 869, DE-OS 1 901 517 und DE-OS 2 801 455 angegeben, eingeführt werden.

Hierbei kann man zum Beispiel Carboxylmethylstärke bei einem pH-Wert grösser als 7 mit einem Farbstoff, der zum Beispiel Sulfatoäthylsulfongruppen (SES), das heisst das Strukturelement $-SO_2-CH_2-CH_2-OSO_3H$ aufweist, zur Reaktion bringen. Dem Ansatz fügt man zweckmässigerweise noch ein Alkaliphosphat bei. Nach mehreren Stunden bei Raumtemperatur wird der Ansatz neutral gestellt und der Farbstärkekomplex isoliert.

Die Verwendung von Verbindungen der Formel I für Indikatorpapiere, -pulver und -folien führt zu Produkten, die sich besonderes dadurch auszeichnen, dass sie bei Berührung mit Wasser nicht ausbluten.

Zur Herstellung nichtblutender Indikatorpapiere werden in der DE-AS 1 256 445 Moleküle mit chromophoren Gruppierungen und reaktiven Zentren, wie beispielsweise dem Oxethylsulfonsäurerest, chemisch an Cellulose oder regenerierte Cellulose gebunden.

Nach diesem Verfahren erhält man keine Papiere, die den Anforderungen der Praxis genügen (DE-OS 1 698 247). Weiter sind solche Indikatorpapiere erfahrungsgemäss nur schwierig herzustellen, da Cellulose erst umständlich in der Masse gefärbt werden muss, um anschliessend daraus Papiere herzustellen, was zu einer Belastung der Umwelt durch grosse Abwassermengen führt. Ausserdem ist eine wirtschaftliche Indikatorpapierproduktion nur dann gegeben, wenn grosse Mengen an Papieren hergestellt werden.

In der DE-AS 2 436 257 wird ein Verfahren beschrieben, bei dem die eingesetzten Indikatoren substantive Eigenschaften haben, dass heisst, die chemischen Verbindungen ziehen auf Baumwolle oder ähnliche natürliche Trägermaterialien so auf, dass sie nicht mehr ausbluten. Diese Papiere haben Nachteile.

Papiere, die entsprechend diesem Verfahren hergestellt werden, sind nur bedingt ausblutecht. Besonders in den stark basischen Bereichen über pH 10 neigen sie verstärkt zum Ausbluten. Zu ihrer Herstellung müssen in aufwendiger Weise spezielle Chemikalien synthetisiert werden, die zum Teil nur in belästigenden Lösemitteln wie Pyridin oder auch Salzsäure gelöst werden können. Auch diese Indikatorpapiere müssen über das umständliche Färben von Cellulose in der Masse und Ausgiessen zu Blättern hergestellt werden.

Ein Ziel der vorliegenden Erfindung war es einen einfachen Weg zur Herstellung nichtblutender Indikatorpapiere aufzuzeigen. Diese Papiere soll-

ten dazu in beliebiger Vielfalt auch in kleinen Mengen und ohne grossen Aufwand herzustellen sein.

Zur Lösung der gestellten Aufgabe werden Verbindungen der Formel I verwendet. Diese Verbindungen können gegebenenfalls nach einer in der Chemie üblichen Methoden gereinigt, als eine wässrige, optisch klare Lösung auf einen Träger, vorzugsweise ein saugfähiges Material, besonders Cellulose, aufgetragen werden. Nach dem Trocknen haftet der Farbkomplex so fest, dass kein Bluten der Farbstoffe in wässrigen oder organisch-wässrigen Lösungen zu beobachten ist.

Geeignete Verbindungen der Formel I können beispielsweise dadurch erhalten werden, dass pH-Indikator-Farbstoffe mit einem Reaktivanker mit einer Stärke umgesetzt werden. Eine solche Verbindung kann auf eine Trägermatrix, beispielsweise Cellulose, aufgetragen werden. Der Einfachheit halber wird man ein Papier in die wässrige Lösung der Verbindung eintauchen oder die Imprägnierlösung in bekannter Weise mittels einer Feder auf die Matrix auftragen. Nach einer abschliessenden Trocknung ist das imprägnierte Papier gebrauchsfhäig. Die Vorteile eines so hergestellten Indikatorpapiers sind beispielsweise darin zu sehen, dass es in beliebiger, auch kleiner Menge herstellbar ist, und dass aufwendige Anlagen zur Papierherstellung nicht benötigt werden.

Die Verbindungen der Formel I können auch als festes Pulver isoliert werden, welches dann beliebig weiterverarbeitbar ist. So können nach Zusatz von Binde- und Klebemitteln zum Beispiel Indikatortabletten hergestellt und durch Einbetten des Pulvers in eine Kunststoffmatrix Indikatorfolien erhalten werden. Sie sind demnach allgemein unter Verwendung künstlicher oder natürlicher flächenbildender Polymere zur Herstellung von Folien und Tabletten geeignet.

Die neuen Indikatorpapiere können beispielsweise als pH-Indikatorpapiere oder als Schnelldiagnostika zur Untersuchung von Lösungen dienen. So können entsprechend dem in der DE-OS 2 141 487 beschriebenen Verfahren Verbindungen der Formel I erhalten werden, die beispielsweise zum Nachweis von Nitrit, Bilirubin und Urobilinogen in biologischen Flüssigkeiten oder als Oxidationsindikator eingesetzt werden können.

Sie sind allgemein verwendbar zur Bestimmung chemischer Kenngrössen in Flüssigkeiten, vor allem in sogenannten Schnelldiagnostika.

Die nachstehenden Beispiele sollen die Erfindung näher erläutern:

Beispiel 1

Herstellung einer carboxymethylierten Farb-Stärke

In 100 ml Wasser werden 5 g Natriumchlorid, 0,1 Tetranatriumdiphosphat, 1 g eines der unten genannten reaktiven Farbstoffe und 2,5 g CM-Stärke (Substitutionsgrad: 0,13) gelöst.

Dazu werden 1,5 ml 10 n Natronlauge gegeben.

Die Lösung wird 2 Stunden bei Zimmertemperatur gerührt, sodann mit 100 ml Phosphatpuffer (70 mmol, pH 7) verdünnt, die Farbstärke mit 800 ml Methanol ausgefällt und dreimal mit jeweils 100 ml Methanol gewaschen. Das erhaltene getrocknete Produkt wird, sofern nötig, zerkleinert. Andere carboxymethylierte sowie hydroxyalkylierte Farb-Stärken können analog hergestellt werden. Geeignete reaktive Farbstoffe sind zum Beispiel Remazol®-, Cibachron®-, Drimaren®-, Procion®- und Lewafix®-Farbstoffe.

Beispiel 2

Herstellung eines Testpapiers

a) 200 $cm^2$ Indikatorrohpapier (140 $g/m^2$) werden mit folgender Lösung getränkt und getrocknet: 2,5 g CM- (oder HA-) Farb-Stärke, 2,5 g Natriumchlorid und 0,1 g Natriumazid, gelöst in 100 ml Phosphatpuffer (70 mmol, pH 7).

Die erhaltenen Papiere können wie folgt genutzt werden:

Auf ein Stück des Testpapiers wird ein Tropfen einer amylasehaltigen Probe aufgetropft, ca. 2 min einwirken gelassen, das Papier mit destilliertem Wasser abgespült und abgetupft. Der Amylasegehalt lässt sich am Grad der Farbauswaschung (-bleichung) ablesen.

b) Die Lösung des Beispiels 2a) lässt sich auch auf Papier in Form eines oder mehrerer Striche aufbringen und das Papier quer zur Richtung der Striche in handliche (ca. 5–10 mm breite) Streifen schneiden. Ein so erhaltener Streifen wird mit einem Ende in die amylasenhaltige Probe eingetaucht. Die Flüssigkeit chromatographier durch den oder die querverlaufenden Farb-Stärke-Zonen und nimmt dabei den durch Amylaseangriff freigesetzten Farbstoff mit sich. Unterschiedliche Amylasegehalte lassen sich entweder durch unterschiedliche Farbtiefe der weiterchromatographierenden Flüssigkeit ablesen (Tabelle I) oder durch die Anzahl der abgelösten Striche (Tabelle II).

Tabelle I (SCE = Street-Close-Einheiten)

| 0 | 25 | 50 | 100 | 200 | 400 | 800 | 2400 | 6400 |
|---|---|---|---|---|---|---|---|---|
| SCE/dl | | | | | | | | |
| 0 | 1 | 1 | 2 | 3 | 4 | 5 | 6 | 7 |

Die ansteigenden Ziffernfolge gibt die voneinander unterscheidbaren Farbintensitäten in der chromatographierenden Flüssigkeit nach Durchtritt der Farb-Stärke-Zone an.

Tabelle II (SCE = Street-Close-Einheiten)

| 0 | 25 | 50 | 100 | 200 | 400 | 800 | 1600 | 3200 | 6400 |
|---|---|---|---|---|---|---|---|---|---|
| SCE/dl | | | | | | | | | |
| 10 | 10 | 10 | 10 | 8 | 6 | 4 | 2 | 1 | – |

Die Zahlen geben die Zahl der noch vorhandenen Linien nach 5 min Reaktionszeit an.

Beispiel 3

Die Verwendung eines Accelerators oder Inhibitors gestattet die Einstellung eines nachzuweisenden Aktivitätsbereiches.

a) Eine Lösung gemäss Beispiel 2 a) wird mit 5 g/l Monflor®51 (Serva 29 842, Heidelberg) und 2 g/l Albumin als Acceleratoren versetzt und ein Papier analog Beispiel 2 b) hergestellt. Das Testergebnis zeigt Tabelle III.

Tabelle III

| 0 | 25 | 50 | 100 | 200 | 400 | 800 | 1600 | 3200 | 6400 |
|---|---|---|---|---|---|---|---|---|---|
| SCE/dl | | | | | | | | | |
| 10 | 10 | 10 | 9 | 7 | 5 | 3 | 1 | – | – |

Die Zahlen geben die Zahl der noch vorhandenen Linien nach 5 min Reaktionszeit an.

b) In einer Lösung gemäss Beispiel 1 werden 50 mg/l eines Amylaseinhibitors, gewonnen entsprechend DE-AS 2 003 934 gelöst. Die Lösung wird analog Beispiel 3 a) verarbeitet. Testergebnisse zeigt Tabelle IV.

Tabelle VI

| 0 | 25 | 50 | 100 | 200 | 400 | 800 | 1600 | 3200 | 6400 |
|---|---|---|---|---|---|---|---|---|---|
| SCE/dl | | | | | | | | | |
| 10 | 10 | 10 | 10 | 10 | 9 | 8 | 6 | 5 | 3 |

Die Zahlen geben die Zahl der noch vorhandenen Linien nach 5 min Reaktionszeit an.

Beispiel 4

Herstellung von pH-Papieren

Nichtblutende pH-Papiere werden entsprechend dem Beispiel 1 erhalten, wenn die in der folgenden Tabelle aufgeführten Indikatoren eingesetzt werden.

| Indikator | Farbumschlag | pH-Bereich |
|---|---|---|
| Remazolbrillantorange BR | orange-blaurot | 10-12 |
| Cibachromorange PP | orange-braun | 10-13 |
| Remazolgelb FG | gelb-braun | 10-14 |
| 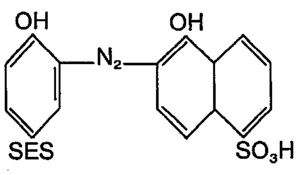 | rot-blau<br>blau-orange | 5-8<br>10-13 |
| 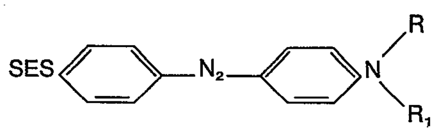 | gelb-rot | 3-1 |
| 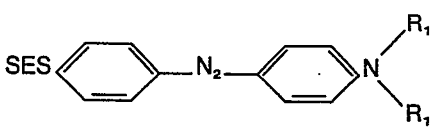 | orange-karminrot | 5-2 |

SES: $-SO_2-CH_2-CH_2-OSO_3H$

R: $-CH_2-CH_3$

$R_1$: $-(CH_2)_2OSO_3H$

Durch die zusätzliche Verwendung von Aluminiumsulfat oder Netzmitteln (z.B. quartäre Ammoniumverbindungen) kann der Gebrauchswert der Papiere noch verbessert werden.

Beispiel 5

Indikatorpapiere für klinischchemische Nachweise

Entsprechend dem Beispiel 1 wird ein aromatisches Amin, beispielsweise 2-Bromanilin, über eine Vinylsulfongruppe an eine carboxyalkylierte oder hydroxyalkylierte Stärke gebunden. Der aufgereinigte Indikatorstärkekomplex wird anschliessend diazotiert. Das gebildete Diazoniumsalz wird zusammen mit Puffern und Stabilisatoren auf ein Chromatographie-Papier aufgetragen. Dieses ist nach dem Trocknen für den Nachweis von Bilirubin in Körperflüssigkeiten geeignet.

Analog werden Papiere zum Nachweis von Urobilinogen in Körperflüssigkeiten hergestellt.

Ein Anilin-Stärkekomplex kann weiterhin zusammen mit organischen Säuren und einen Kuppler wie β-Naphthylamin auf ein Chromatographie-Papier aufgetragen werden. Dieses Papier kann dann als Mittel zum Nachweis von Nitrit verwendet werden.

**Patentansprüche**

1. Verwendung einer Stärkeverbindung der allgemeinen Formel I

$$\begin{array}{c} RO\text{–[Glucoserest]}_n\text{–}OR \\ | \\ (OR)_{3n} \end{array} \qquad (I)$$

worin

n = 100–12 500 und

R Wasserstoff, eine Carboxyalkyl- oder Hydroxyalkylgruppe mit 1–5 Kohlenstoffatomen im Alkylrest und gegebenenfalls eine nachweisbare Molekülgruppe bedeuten, wobei die (3 n + 2) R-Reste statistisch verteilt sind, jedoch so, dass mindestens ein R eine Carboxy- oder Hydroxyalkylgruppe ist, für Indikatorpapiere, -pulver oder -folien.

2. Verwendung einer Stärkeverbindung gemäss Formel I in Anspruch 1, in der

n = 350–12 500 und

R Wasserstoff, eine Carboxy- oder Hydroxyalkylgruppe mit 1–3 Kohlenstoffatomen im Alkylrest und gegebenenfalls eine nachweisbare Molekülgruppe sind.

3. Verwendung einer Stärkeverbindung gemäss Formel I in Anspruch 1, in der

n = 600–6000 und

R Wasserstoff, eine Carboxy- oder Hydroxyalkylgruppe mit 1–2 Kohlenstoffatomen im Alkylrest und gegebenenfalls eine nachweisbare Molekülgruppe sind.

**Claims**

1. Use of a starch derivative of the general formula I

$$\begin{array}{c} RO\text{–[glucose residue]}_n\text{–}OR \\ | \\ (OR)_{3n} \end{array} \qquad I$$

in which

n denotes a number from 100 to 12 500 and

R denotes hydrogen, a carboxyalkyl or hydroxyalkyl group with 1–5 carbon atoms in the alkyl radical or optionally a detectable molecular group, the (3n + 2) R radicals being randomly distributed, but at least one R being a carboxyalkyl or hydroxyalkyl group in indicator papers, indicator powders or indicator foils.

2. Use of a starch derivative according to formula I in claim 1, in which

n is a number from 350 to 12 500 and

R is hydrogen, a carboxyalkyl or hydroxyalkyl group with 1–3 carbon atoms in the alkyl radical or optionally a detectable molecular group.

3. Use of a starch derivative according to formula I in claim 1, in which

n is a number from 600 to 6000 and

R is hydrogen, a carboxyalkyl or hydroxyalkyl group with 1–2 carbon atoms in the alkyl radical or optionally a detectable molecular group.

**Revendications**

1. Utilisation d'un composé d'amidon de formule générale I

$$\begin{array}{c} RO\text{–[radical glucose]}_n\text{–}OR \\ | \\ (OR)_{3n} \end{array} \qquad (I)$$

dans laquelle

n est un nombre de 100 à 12 500, et

R représente l'hydrogène, un groupe carboxyalkyle ou hydroxy-alkyle ayant de 1 à 5 atomes de carbone dans la partie alkyle et éventuellement un groupe moléculaire détectable, les (3n + 2) groupes R étant alors statistiquement distribués, mais de telle sorte qu'au moins un groupe R est un groupe carboxyalkyle ou hydroxyalkyle, pour des indicateurs sous forme de papiers, poudre ou films.

2. Utilisation d'un composé d'amidon selon la formule I de la revendication 1, dans lequel

n est un nombre de 350 à 12 500 et

R représente l'hydrogène, un groupe carboxy- ou hydroxyalkyle ayant de 1 à 3 atomes de carbone dans la partie alkyle et éventuellement un groupe moléculaire détectable.

3. Utilisation d'un composé d'amidon selon la formule I de la revendication 1, dans lequel

n est un nombre de 600 à 6000 et

R représente l'hydrogène, un groupe carboxy- ou hydroxyalkyle ayant 1 ou 2 atomes de carbone dans la partie alkyle et éventuellement un groupe moléculaire détectable.